# EUROPEAN PATENT SPECIFICATION

(11) **EP 2 353 552 B1**
(45) Date of publication and mention of the grant of the patent: **03.07.2013**
(21) Application number: 10152711.7
(22) Date of filing: 04.02.2010
(51) Int. Cl.: A61F 2/95, A61F 2/94

(54) **System for deploying or retracting a prosthesis**
System zur Anwendung oder Herausnahme einer Prothese
Système pour déployer ou rétracter une prothèse

(43) Date of publication of application: 10.08.2011
(73) Proprietor: Joline GmbH & Co. KG, 72379 Hechingen (DE)
(72) Inventor: Haag, Wolfgang, 46487 Wesel (DE); Nakath, Thorsten, 46487 Wesel (DE); Seidenberger, Dieter, 72379 Hechningen (DE); Steegers, Anselm, Dr., 72108 Rottenburg (DE)
(74) Representative: Witte, Weller & Partner

(56) References cited:
- EP-A1- 0 943 300
- EP-A1- 1 872 749
- US-A1- 2003 114 909
- US-A1- 2006 135 963
- US-A1- 2006 276 873

## Description

The present invention relates to a system for deploying and/or retracting a tubular prosthesis in/from a biliary or pancreatic duct of a human body, the system comprising an elongated outer or pusher tube having a proximal and a distal end, an elongated inner or carrier tube, having a proximal and a distal inner tube portion, the inner tube being longer than the outer tube, being partially disposed within the outer tube, and being movable with respect to the outer tube, wherein the outer tube and the inner tube are concentrically arranged with respect to one another.

Prostheses deployment devices and systems of this kind are known in the art, and are used to introduce and release prostheses such as stents, implants etc. within body lumina. The placement of tubular prostheses in body lumina, such as blood vessels or ducts, is desirable in the treatment of a number of medical conditions. For example, where a vessel or a passage is obstructed it may be desirable to insert a prosthetic tube within the vessel to provide for an unhindered passageway through the respective body lumen; an example for such a condition is the constriction or stenosis of the bile duct or the pancreatic duct. Treatment of this condition is - in most cases - achieved by inserting a prosthesis in form of a tube into the bile or pancreatic duct to maintain the flow passage open through the bile duct. Several numbers of techniques are known to place the prosthesis in the bile or pancreatic duct, which involve insertion of catheters, probes and various other devices to guide, manipulate and position the prosthesis. Generally, the systems comprise a catheter carrying the prosthesis and, at least in some devices, a sheath or tube covering the prosthesis as well as a pusher tube, wherein for releasing the prosthesis the sheath is being retracted as soon as the prosthesis is positioned at the location to be treated, and wherein the pusher tube serves to hold the prosthesis at the desired location against the retracting forces exercised by the sheath or the inner carrier tube.

Biliary or pancreatic prostheses which are applied these days to treat duct obstruction generally represent PTFE (polytetrafluorethylene) tubes having a cross-section of about 2 to 4 mm and a length of about 5 to 20 cm. They are implanted by means of a system generally consisting of two concentrically positioned tubes, an inner tube and an outer tube, wherein the inner tube extends from the outer tube. A prerequisite for a successful treatment is the stable and enduring placement of the prosthesis since a displacement or migration of the biliary/pancreatic prosthesis may cause severe lesions of the bile duct, other vessels or even organs, and may even lead to a perforation of the liver or the pancreas. Thus, in order to avoid a displacement, the prostheses often include retaining means at one or both of their ends, such as flaps, barbs, pigtail loops, etc. Also, it is often necessary to remove an introduced biliary prosthesis, e.g. in case where the primary location has proven to be suboptimal. Furthermore, in medical practice, the prostheses have to be replaced on regular basis due to the fact that a lumen of the prosthesis itself got obstructed, which in general is necessary each 3 months.

In this connection, WO 99/08740 discloses a drainage catheter delivery system for positioning a drainage catheter or stent within a body cavity, including a guide catheter, a push catheter, a drainage catheter and a retention device. The guide catheter slidably receives the push catheter and the drainage catheter. The push catheter is axially slid along the guide catheter to move the drainage catheter away from a proximal end of the guide catheter.

US 4,699,611 discloses a device and technique for percutaneous placement of a tubular prosthesis. The prosthesis consists of a stent having lobes on a Mallecot tip, which are gripped by the distal portion of a carrier sheath so that the lobes will be tensioned and will be drawn longitudinally into a radially collapsed configuration to define a substantially continuous tubular shape along the full length of the stent. The distal portion of the carrier sheath, thus, holds the stent securely during insertion; it is formed by slitting it longitudinally and curling the slit edges with one another, defining a smaller outer diameter then the more proximal portion of the carrier sheath. By advancing a dilator through the carrier sheath, the distal end is forced outwardly into frictional engagement with the lumen of the stent. Further, EP 0 943 300 A1 discloses an endoprosthesis delivery device comprising an axially extending inner tube surrounded by an outer sheath comprising remote-activated retaining means able at will to maintain the endoprosthesis in a given axial relationship relative to the inner tube. Also, the endoprosthesis can be withdrawn and replaced in a more suitable position. US 2006/276873 A1 discloses the preamble of appended claim 1.

With the systems and prostheses known in the art, the retraction of the prostheses, which is - in the most cases - necessary after about three months, is often involved with a surgical intervention, since the flaps or loops provide for a stable and secure positioning of the prosthesis which may otherwise not be retrieved from the duct. By means of the pusher the prosthesis can be moved distally only. Also, by means of the flaps, the prosthesis may only be moved in a distal direction, i.e. in the direction opposite to the operator, and may not be moved back and forth, which movement may be necessary to (re)position the prosthesis more precisely.

In view of the above, it is an object of the present invention to provide an improved prosthesis delivery and retraction system by means of which it is possible to securely position the prosthesis in the respective duct, and to optionally move it back and forth in the duct, and to provide for a system by means of which a prosthesis may be retracted, also.

According to the invention, this and other objects of the invention are achieved by a system as mentioned in the appended claims, wherein in addition the intermediate portion of the inner carrier tube comprises at least one inflatable element which is convertible from an inflated state to a deflated state, and which has the shape of a short tube with the two endings of the short tube being mounted on the inner tube's intermediate portion, wherein for positioning the prosthesis within a duct or for retracting the prosthesis from a duct, the inflatable element is in the inflated state to fixate the prosthesis in respect of the inner carrier tube, and wherein for releasing the prosthesis in the duct at a desirable location the element is operable to be converted into the deflated state. The system according to the invention may optionally further comprise a tubular prosthesis positionable on the inner tube.

The object underlying the invention is completely solved in this way.

With the system according to the invention a tool is provided, by means of which an operator handling the system can securely move a prosthesis, even back and forth, in a duct until the correct or desired position in the duct has been found. The system is easy to handle and comprises a straightforward design, which allows a cost-effective and economic manufacture.

The system according to the invention may be applied in mammals, in particular in humans, to treat obstruction or other conditions of ducts in their body. With the inner tube comprising an inflatable element on the end carrying the prosthesis, the prosthesis may be fixed relative to the inner tube upon inflating the inflatable element, and can, thus, by movement of the inner tube be moved backwards and forwards in the duct to find the correct position in the duct.

The system according to the invention is, thus, particularly suitable for application within the bile duct or the pancreatic duct to deploy or to retract/reposition biliary or pancreatic prostheses.

Throughout this specification, the term "distal" shall mean furthest from the operator handling the system, and "proximal" shall mean nearest to the operator handling the system. Thus, the term "proximal", with respect to a tube, describes the direction towards an operator handling the tube, while the expression "distal" describes the direction towards the tube end facing away from the operator. Thus, the proximal end of a structure represents the end which is nearer to the operator handling the structure, and the distal end represents the end which is opposed to the proximal end and, thus, farther away from the operator. In this connection, the "distal" end of the outer pusher tube is the end of the tube that lies further from the operator than the other end, which is designated "proximal" end. Same applies for the ends or portions of the inner carrier tube.

The expression "inflatable" shall in this context mean the feature of an object or element that can be inflated with a gas, such as oxygen, or a liquid. In particular, any gas or liquid that is physiologically harmless for the mammalian body may be used. Such gases and liquids are applied in several other medical and surgical applications, in particular in connection with inflating elements, and are known as such in the art. The inflatable element according to the invention has in its deflated state, a configuration that lies smooth against the outer surface of the inner tube, i.e. without forming crinkles or the like, so that upon placing the prosthesis on the inner tube and upon removing the inner tube from the prosthesis substantially no or only little friction occurs.

The system according to the invention as disclosed in the appended claims comprises two concentrically positioned tubes, wherein the inner tube is longer than the outer pusher tube and extends with its distal portion out from the distal opening of the outer tube. The portion of the inner carrier tube, that extends or projects from the distal end of the outer pusher tube, carries at least one inflatable element. The inflatable element is, thereby, mounted on or about the outer periphery of a portion of the inner tube; the inflatable element may be mounted on the inner tube's outer surface in a circumferential manner, i.e. wholly encompassing or surrounding the inner tube's outer surface over a certain portion's distance, or only partially, so that in the latter case the periphery of a portion of the inner tube is not fully covered by the inflatable element. The tubular prosthesis is arranged over at least a portion of the inner carrier tube comprising the inflatable elements, such, that the inflatable element is being positioned between the prosthesis and the inner carrier tube. Upon inflating of the inflatable element, the walls of the inflatable element, which in the deflated state adapt, e.g., to the form of the outer (circumferential) surface of the inner carrier tube, are pressed outwardly, i.e. against the tubular prosthesis positioned on the inner carrier tube over the inflatable element, thereby frictionally fixating the prosthesis. In this configuration, the prosthesis may be introduced into the duct and repositioned until the correct location in the duct has been found. Subsequently, the inflatable element is deflated whereupon the prosthesis is no longer fixed relative to the inner carrier tube and can be released by retracting the inner tube whilst holding up the outer tube against the proximal end of the prosthesis, leaving the prosthesis securely positioned in the duct. Thus, the outer tube functions as a pusher.

The inflatable element is mounted on the inner tube, or rather on the outer surface of the inner tube, by means of, e.g. shrinking, bonding, gluing, welding etc. Thus, the inflatable element has - prior to its mounting on the inner tube - the form of a short inflatable tube comprising two endings by means of which it is mounted/attached to the inner tube.

For explanting a prosthesis, the system according to the invention is introduced with its inner tube into the tubular prosthesis, such, that the deflated inflatable element located on the surface of the inner tube is positioned - at least partially - beneath, or rather: within the tubular prosthesis. Upon inflating the inflatable element, the walls of the inflatable element are pressed against the prosthesis, thereby fixing the prosthesis relative to the inner tube. Subsequently, the system can be retracted and the prosthesis can be removed from the duct.

According to a preferred embodiment of the system of the invention, the inflatable element is a balloon.

Inflatable balloons are commonly used in connection with medical instruments such as, e.g. balloon catheters, which comprise a "balloon" at their tips which is used during a catheterization procedure to enlarge a narrow or occluded opening or passage within the body of a mammal. Suitable materials for the balloon are, thus, known to those skilled in the art, as well as gases and fluids the inflatable element/balloon may be inflated with. Suitable materials include, e.g., polyether block amide, polyamide, polyurethane.

According to a preferred embodiment, the balloon comprises an elongated shape. Thus, in this embodiment, the balloon is designed such, that its wall in its deflated state and when surrounding the outer surface of the inner tube adapts the form of the inner tube over a certain distance. In the inflated state, the portion of the balloon that is not mounted/attached to the inner tube, i.e. the portion lying between the two endings, "detach" from the inner tube's surface, thereby forming a gas- or liquid-filled space between the surface of the inner tube and the wall of the inflatable element/balloon. Thus, in the inflated state, the walls of the balloon press against the prosthesis located over the inflatable element, thereby fixating the prosthesis relative to the inner tube. The balloon comprising an "elongated" shape thus has itself the form of a short tube.

The inflatable element may be inflatable by a gas or a liquid that is being fed into the element's inner space via one or more openings in the surface of the inner tube arranged beneath the inflatable element. Thus, the inflatable element comprises a tubular body which is attached to the outer surface of the inner carrier tube only via its two end portions, whereby its intermediate portion lying between the two end portions is free. Once the prosthesis has been correctly positioned, the gas or liquid in the inflatable element's body is removed again via the openings in the inner tube. For this purpose, the system also comprises an inflation/deflation lumen, which e.g. might be a portion of the inner tube thereby building a double lumen tube, i.e. the lumens of the inner tube are located lateral or side by side, or the inflation lumen is realized by means of a further tube positioned within the inner tube concentrically, respectively.

According to yet another embodiment of the system according to the invention, a first inflatable element is arranged on the inner carrier tube at the intermediate portion thereof, such, that it is located substantially adjacent to the distal end of the outer tube.

This feature is of advantage in that for introducing the prosthesis, it may be positioned on the inner carrier tube right next to the distal end of the outer pusher tube, i.e. on the portion of the inner carrier tube just extending out from the outer tube. Thus, when retracting the tube and holding up against the prosthesis, an accidental dislocation of the prosthesis can be avoided.

According to yet another embodiment, the inner carrier tube comprises a second or more inflatable element(s), the second inflatable element(s) being located at the outermost portion of the distal portion of the inner carrier tube.

This embodiment is particularly suitable for explanting a prosthesis, since with the inflatable element being arranged on the outermost portion of the distal portion of the inner carrier tube, it is sufficient to introduce the inner tube only partially into the duct, i.e. only with the distal portion or tip carrying the inflatable element, thereby minimizing the risk of duct injuries.

Thus, according to the invention, different embodiments of the system according to the invention are provided, which may be used according to their specific appliance: Systems which comprise one inflatable balloon that is arranged on the outer surface of the inner tube adjacent to the distal end of the outer tube, are particularly suitable for introducing a prosthesis, whilst a system having an inflatable element arranged on the outer surface of the inner tube's very distal end (not part of the invention, is not combined with the previous system), is particularly suitable for explanting a prosthesis. Also, systems which, arranged on the inner tube, comprise both, one (or more) inflatable elements adjacent to the distal end of the outer pusher tube and one or more inflatable elements on the outermost portion of the distal end of the inner tube.

In this connection and according to another preferred embodiment of the invention, the two or more inflatable elements can be inflated independently or together.

According to another aspect, the system according to the invention comprises at least one radiopaque marker element on at least one of the following: prosthesis, outer pusher tube, inner carrier tube.

By means of the radiopaque element(s) it is possible to introduce the system and/or to release the prosthesis under X-ray observation.

Generally, the systems according to the invention are suitable to be applied endoscopically; typically, an endoscope is introduced into the duct and its distal end is being positioned in proximity to the desired anatomical area to be treated. Subsequently, or optionally after dilation of the stricture, a guide wire is inserted, and a guide catheter is placed over the guide wire to provide instrumental access to the duct. In a next step, the system according to the invention is placed into the guide catheter and moved into the duct to the location or area to be treated. Once properly positioned, the inflatable element is deflated, the prosthesis, thus, released, and the system is removed from inside of the guide catheter.

Thus, according to another aspect of the present invention, the system further comprises a guide wire, being locatable within the inner carrier tube.

In a preferred embodiment, the system comprises on each of the proximal portion of the inner carrier and the outer pusher tube, respectively, a grip or handle member.

According to yet another embodiment, the grip members can be fixated with respect to one other and released from one another again.

This measure is of advantage since for introducing the system - as well as the prosthesis arranged thereupon - the grips can be fixed, thereby fixing the inner tube relative to the outer tube, and avoiding a displacement of the inner tube relative to the outer tube. As soon as the prosthesis is correctly positioned, the inflatable element is deflated and the grips are detached again, in order to be able to move the inner tube outwards and relative to the outer tube.

The system according to the invention, as described above as well as to be explained in more detail below, may be used and suitable for deployment in the bile duct and the pancreatic duct. Those skilled in the art will realize that the system described herein may be applied for all different appliances and interventions in the body of a mammal, in particular a human body, where a duct has to be treated to receive a prosthesis.

With the system according to the invention, a method for deployment of a hollow, tube-like prosthesis within a biliary or pancreatic duct of a human body can be performed, the method comprising the steps of:
- providing a system comprising an elongated outer tube having a proximal and a distal end, and an elongated inner tube, having a proximal, a distal, and an intermediate inner tube portion, the inner tube being partially disposed within the outer tube and being movable with respect to the outer tube and extending from the distal end of the outer tube, wherein the outer tube and the inner tube are concentrically arranged with respect to one another, and wherein the inner tube comprises at least one inflatable element which is convertible from an inflated state to a deflated state;
- disposing - at least partially over the inflatable element - a prosthesis on the distal portion of the inner tube, adjacent to the distal end of the outer tube;
- inflating the inflatable element for fixing the prosthesis relative in respect to the inner tube;
- inserting the prosthesis, the inner tube and the outer tube within the duct; and
- deflating the inflatable element at a desired location within the bile or pancreatic duct to release the prosthesis from the inner tube and to implant the prosthesis within the bile or pancreatic duct.

The method is easily to apply and requires only few steps to deploy the prosthesis in the duct.

In addition, the system according to the invention can also be used in a method for retracting or repositioning of a hollow, tube-like prosthesis from a bile duct of a human body comprising the steps of:
- providing a system comprising an elongated outer tube having a proximal and a distal end, and an elongated inner tube, having a proximal, a distal and an intermediate inner tube portion, the inner tube being partially disposed within the outer tube and being movable with respect to the outer tube, wherein the outer tube and the inner tube are concentrically arranged with respect to one another, and wherein the inner tube comprises at least one inflatable element which is convertible from an inflated state to a deflated state;
- inserting the inner tube and the outer tube within the bile duct and the inner tube within the lumen of the prosthesis, such, that the prosthesis is disposed about the inflatable element of the inner tube;
- inflating the inflatable element to fixate the prosthesis in respect to the inner tube; and
- retracting or repositioning the prosthesis fixed to the inner tube by means of the inflated inflatable element from the bile duct by moving the inner tube in the direction of the operator.

With the system according to the invention, prostheses not only may be deployed or introduced into the duct of a body, but also prostheses already present in a duct, can easily be removed without necessitating a major surgical intervention.

It is understood that the features described above and those still to be specified below fall within the scope of the present invention not only in the respectively specified combinations, but also in other combinations or on their own.

Several embodiments of the invention are illustrated in the figures and explained in more detail in the following description. In the figures:
- Fig. 1: shows a schematic drawing of a perspective view of a system according to the invention (without prosthesis);
- Fig. 2: shows an enlarged schematic view of the system of Fig. 1, along the line A->A', with the prosthesis mounted on the inner tube of the system;
- Fig. 3: shows a cross-sectional view of the system shown in Figs. 1 and 2, at the position B indicated in Fig. 1; and
- Fig. 4: shows an enlarged schematic view of the distal portion of the inner tube of the system as shown in Fig. 1, with the inflatable element being deflated (A), and with the inflatable element being inflated (B).

In the figures, same features of the embodiments shown in the figures are designated with the same reference signs.

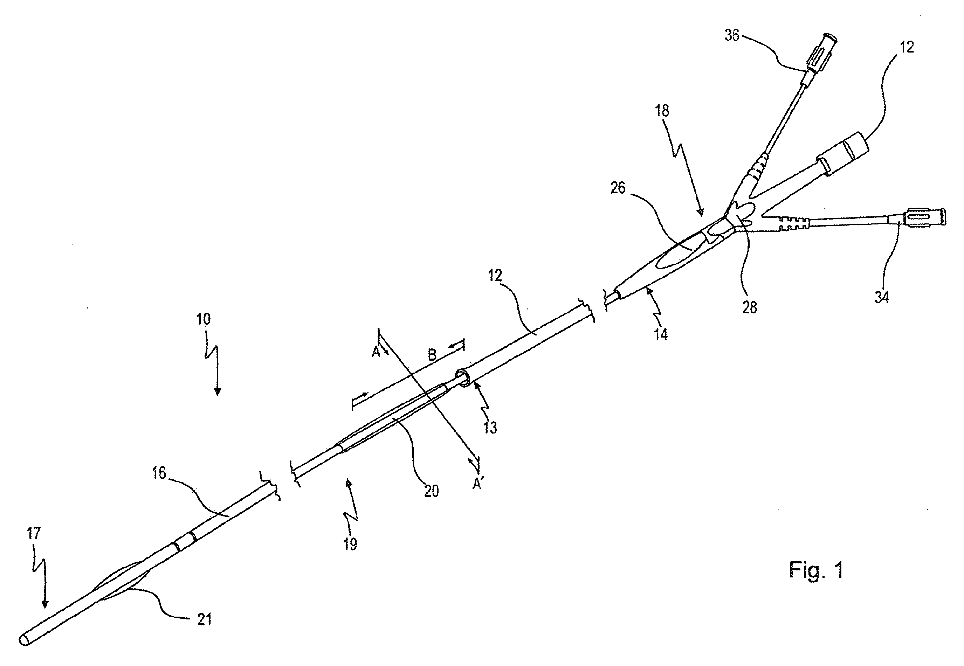

Fig. 1 is a schematic drawing of an embodiment of a system 10 according to the invention. The system 10 comprises an outer pusher tube 12 and an inner carrier tube 16, which are concentrically arranged with respect to one another. The outer tube 12 has a distal end 13 and a proximal end 14. The inner tube 16 extends out from the distal end 13 of the outer tube 12, and is, thus, longer than the outer tube 12. Furthermore, the inner tube 16 is slidably arranged in the outer tube 12, and the inner tube 16 has a distal portion 17, a proximal portion 18, and an intermediate portion 19, located between proximal portion 18 and distal portion 17.

In the embodiment depicted in Fig. 1, inner tube 16 carries an inflatable element 20, the inflatable element 20 being arranged on/about the outer surface of the inner tube's intermediate portion, adjacent to the distal end 13 of outer tube 12. The inflatable element 20 depicted in Fig. 1 has a tube-like shape, with the endings of the short tube - representing the inflatable element - sealingly connected, i.e. glued, shrunk, welded, or other, with/on the outer surface of the inner tube 16. The middle section of the inflatable element is not connected with the outer surface, and may, thus, detach from the surface of the inner tube 16 upon inflating the inflatable element 20 via feeding a gas or liquid via holes or openings 24 present in the surface of the inner tube 16.

Also depicted in Fig. 1 is a second inflatable element 21, mounted in the distal portion 17 of the inner tube 16, and shown in the inflated state.

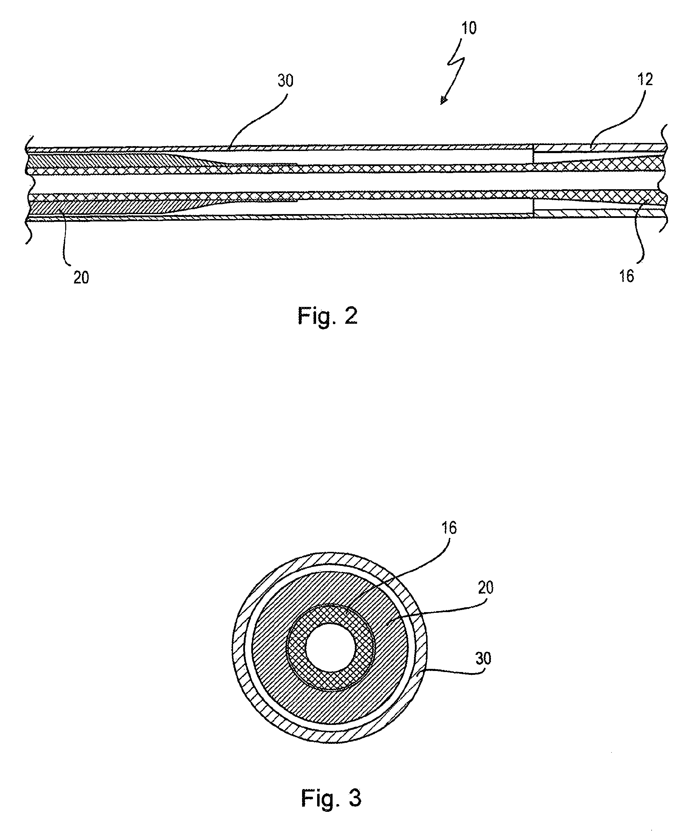

In Fig. 2, an enlarged view of the portion of the system 10 shown in Fig. 1 is depicted, namely the portion where the distal end 13 of outer tube 12 abuts on the proximal end 31 of prosthesis 30. For the sake of clarity, prosthesis 30 is not shown in Fig. 1.

Also shown in Fig. 1 is that on the proximal end 14 of outer tube 12 a grip 26 is arranged by means of which the outer tube may be handled. Further, inner tube 16 comprises at its proximal portion 18 a grip 28 for handling inner tube 16. In the embodiment shown in Fig. 1, the grips are designed such, that they can be interlocked with one another. This is of advantage, so that upon loading the prosthesis 30 on the inner tube 16 and upon introducing the system 10 in the duct of a human body, e.g. via endoscopic means, the inner tube 16 is fixed relative to the outer tube 12 and an undesired dislocation of the prosthesis is avoided. Once the prosthesis 30 is placed at the site to be treated, the inflatable element 20 is deflated, the grips 26 and 28 are detached from one another, and the operator handling the system 10 may retract inner tube 16 and hold outer tube 12 against the prosthesis 30 and against the movement of inner tube 16. Thus, prosthesis 30 is released and securely remains in the duct, whilst the remaining parts of the system 10 may be removed from the duct also.

The grip 28 of inner tube 16 also carries connecting or adapter means, providing access for several lumina, e.g. a guide wire lumen 32, an inflation/deflation lumen 34 and an irrigation/aspiration lumen 36. Via guide wire lumen 32, the system 10 may be threaded over a guide wire (not shown in the figures) which has been previously laid or introduced in the duct. Via inflation/deflation lumen 34, an inflation gas or liquid may be guided to inflatable element 20 for inflating the latter, or the gas/liquid may be removed to deflate it. The irrigation lumen/aspiration 36 is used for feeding an irrigating fluid into the duct or for aspirating material from the duct.

As can be seen in Fig. 2, prosthesis 30 is - at least partially - arranged over the inflatable element 20 mounted on the surface of inner tube 16. Thus, the distal end 13 of outer tube 12 is adjacent to proximal end 31 of prosthesis 30. The inflatable element 20 is, as shown in Fig. 2, inflated and the wall of the inflatable element 20 presses against the inner surface/wall of prosthesis 30, thereby frictionally fixing the prosthesis 30 relative to the inner tube 16. In this configuration, the prosthesis 30 can be introduced into the duct of a body. Once the prosthesis 30 has been correctly placed, the inflatable element 20 is deflated and inner tube 16 can be moved relative to the prosthesis 30 and out of the duct, while prosthesis 30 remains in the duct. The outer tube 12 is held up against the frictional force exerted by the inner tube's 16 removing and against prosthesis 30.

For introducing the prosthesis 30 into a duct, the prosthesis 30 is arranged over the deflated inflatable element 20 by introducing the inner tube 16 into the prosthesis 30, such, that the inflatable element 20 in its deflated state is positioned underneath the prosthesis 30. Once the prosthesis 30 is correctly arranged on the inner tube 16, the inflatable element 20 is inflated.

In Fig. 3, a cross-sectional view of the system 10 depicted in Fig. 1 is shown, with the cut made at positions A to A' as indicated in Fig. 1. As can be seen, the inflatable element 20 is positioned between inner tube 16 and prosthesis 30. The additional lumens of the guide wire lumen 32 and irrigation lumen 36 are, for the sake of clarity, not shown in figures 2 and 3, however, it is to be understood, that these additional lumina are present within the inner tube 16. Also, in Fig. 2, the inflation lumen 34 is not shown. The inflation lumen 34 is, in the embodiment shown in fig. 3, another concentrically arranged tube within the lumen of inner tube 16; alternatively, inner tube 16 and inflation tube 23 may represent a double lumen tube, which is *per se* generally known in the art.

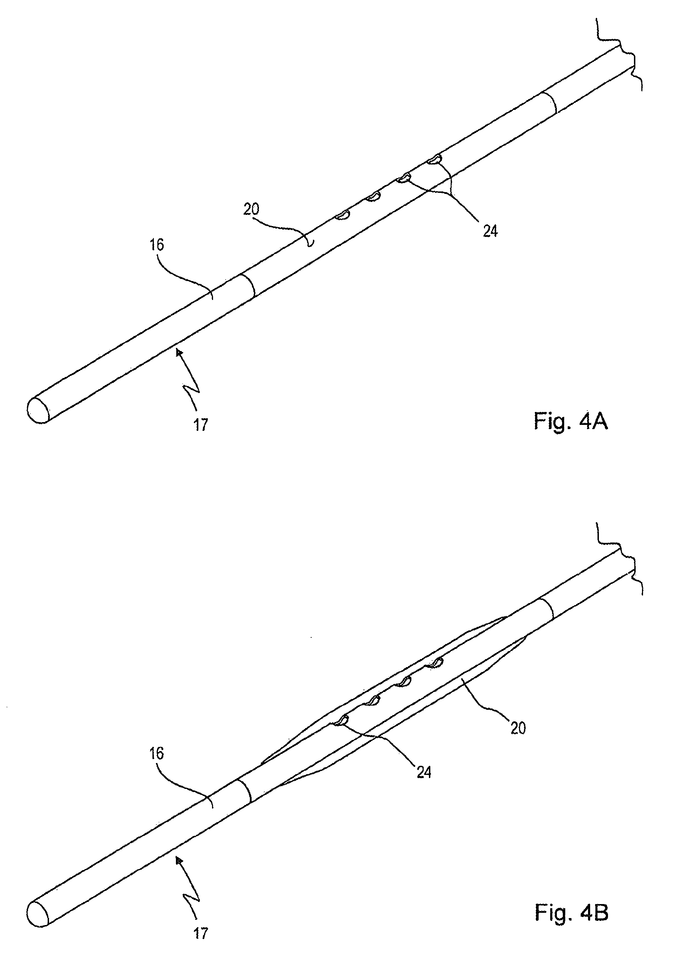

Fig. 4 shows an enlarged view of the distal portion 17 of inner tube 16, with the inflatable element 21 deflated (A) and with the inflatable element 21 inflated (B). As can be seen in Fig. 4, the surface of inner tube 16 comprises openings or holes 24, via which the inflation gas or inflation liquid is fed. The inflation gas or liquid is guided through the inflation lumen 34 to holes/openings 24, and inflates the inflatable element 21.

For explanting a tube-like prosthesis previously placed within a duct of a body, the system 10, or rather mainly its inner tube 16 carrying the deflated inflatable element 21, is introduced in the duct, e.g. via a guide catheter and an endoscope, and guided into the prosthesis, such, that the inflatable element 21 is located at least partially underneath the prosthesis; in a next step, the inflatable element 21 is inflated, whereby the walls of the inflatable element are pressed against the inner walls/surface of the prosthesis. Thus, the prosthesis is frictionally fixated relative to the inner tube 16 and may be removed from the duct via retracting the inner tube from the duct.

The inflatable elements 20, 21 of the system 10 shown in figures 1 to 4 are each designed such, that in their deflated state the material of the inflatable elements 20, 21 tightly lies against the surface of inner tube 16, so as to avoid the formation of crinkles in the material of the inflatable elements 20, 21. In the inflated state, the inflatable elements 20, 21 have an elongated, tube-like form, with the two endings of the tube sealingly mounted on the surface of the inner tube 16. One skilled in the art will recognize that the form and shape of the inflatable element may depend from the shape and design of the prosthesis to be introduced within a duct, and that other forms apart from the tube-like structure are possible.

It is to be understood, that different prosthesis having different sizes/cross-sections can be used and applied in connection with the system 10 according to the invention. The actual size depends on the size and features of the duct of the body and the conditions to be treated. Also, the inflatable element may be designed with respect to the prosthesis to be implanted/inserted.

## Claims

1. System (10) for deploying and/or retracting a tubular biliary or pancreatic duct prosthesis in/from a duct of a mammalian body, the system (10) comprising
- an elongated outer pusher tube (12) having a proximal end (13) and a distal end (14),
- an elongated inner carrier tube (16), having a proximal (18), a distal (17) and an intermediate (19) tube portion, the inner carrier tube (16) being partially disposed within the outer pusher (12) tube and being movable with respect to the outer pusher tube (12), wherein the outer pusher tube (12) and the inner carrier tube (16) are concentrically arranged with respect to one another,
**characterized in that** the intermediate portion (19) of the inner carrier tube (16) comprises at least one inflatable element (20) which is convertible from an inflated state to a deflated state, and which has the shape of a short tube with the two endings of the short tube being mounted on the inner tube's intermediate portion (19), wherein for introducing a prosthesis within a duct or for retracting a prosthesis, the inflatable element (20) is in the inflated state to fixate the prosthesis in respect of the inner carrier tube (16), and wherein for releasing the prosthesis in the duct at a desirable location the inflatable element (20) is operable to be converted into the deflated state.

2. System (10) according to claim 1, wherein the inflatable element (20) is a balloon.

3. System (10) according to claim 2, wherein the balloon comprises in its inflated state an elongated shape.

4. System according to any of claims 1 to 3, **characterized in that** a first inflatable element (20) is arranged on the inner carrier tube (16), such, that it is located substantially adjacent to the distal end (14) of the outer pusher tube (12).

5. System (10) according to claim 4, **characterized in that** the inner carrier tube (16) comprises a second or more inflatable element(s) (21), the second inflatable element(s) (22) being arranged on the distal portion (17) of the inner carrier tube (16).

6. System (10) according to claim 5, **characterized in that** the two or more inflatable elements (20; 21) can be inflated independently or together.

7. System (10) according to one of the aforementioned claims, **characterized in that** it further comprises at least one radiopaque marker element on at least one of the following: prosthesis (30), outer pusher tube (12), inner carrier tube (16).

8. System (10) according to one of the aforementioned claims, **characterized in that** it further comprises guide wire being locatable within the inner tube (16) in a guide wire lumen (32).

9. System (10) according to one of the aforementioned claims, **characterized in that** it further comprises on each of the proximal portion (18) of the inner carrier (16) and the outer pusher (12) tube, respectively, a grip member (26; 28).

10. System (10) according to claim 9, **characterized in that** the grip members (26; 28) can be fixated with respect to each other and released from one another again.

11. System (10) according to any of the aforementioned claims, **characterized in** it further comprises a tubular biliary or pancreatic dcut prosthesis (30) postion-able on the inner tube (16).

## Patentansprüche

1. System (10) zur Freisetzung und/oder Zurückholung einer schlauchförmigen Gallengang- oder Pankreasgang-Prothese in/aus einem Gang eines Körpers eines Säugetiers, wobei das System (10) Folgendes aufweist:
- einen länglichen äußeren Pusherschlauch (12) mit einem proximalen Ende (13) und einem distalen Ende (14),
- einen länglichen inneren Trägerschlauch (16) mit einem proximalen (18), einem distalen (17) und einem mittleren (19) Schlauchabschnitt, wobei der innere Trägerschlauch (16) teilweise innerhalb des äußeren Pusherschlauchs (12) angebracht ist und in Bezug auf den äußeren Pusherschlauch (12) bewegbar ist, wobei der äußere Pusherschlauch (12) und der innere Trägerschlauch (16) konzentrisch in Bezug aufeinander angeordnet sind,
**dadurch gekennzeichnet, dass** der mittlere Abschnitt (19) des inneren Trägerschlauchs (16) zumindest ein inflatierbares Element (20) aufweist, das von einem inflatierten Zustand in einen deflatierten Zustand überführbar ist, und das die Form eines kurzen Schlauchs besitzt, dessen zwei Enden an den mittleren Abschnitt (19) des inneren Schlauchs angebracht sind, wobei zur Einführung einer Prothese in einen Gang oder zur Zurückholung einer Prothese das inflatierbare Element (20) im inflatierten Zustand ist, um die Prothese bezüglich des inneren Trägerschlauchs (16) zu fixieren, und wobei zur Freisetzung der Prothese in dem Gang an einer gewünschten Stelle das inflatierbare Element (20) derart bedienbar ist, dass es in den deflatierten Zustand überführt werden kann.

2. System (10) nach Anspruch 1, wobei das inflatierbare Element (20) ein Ballon ist.

3. System (10) nach Anspruch 2, wobei der Ballon in seinem inflatierten Zustand eine längliche Form aufweist.

4. System (10) nach einem der Ansprüche 1 bis 3, **dadurch gekennzeichnet, dass** ein erstes inflatierbares Element (20) auf dem inneren Trägerschlauch (16) derart angeordnet ist, dass es im Wesentlichen benachbart zum distalen Ende (14) des äußeren Pusherschlauchs (12) zu liegen kommt.

5. System (10) nach Anspruch 4, **dadurch gekennzeichnet dass** der innere Trägerschlauch (16) ein zweites oder mehrere inflatierbare Elemente (21) aufweist, wobei das /die zweiten inflatierbaren Elemente (22) auf dem distalen Abschnitt (17) des inneren Trägerschlauchs (16) angeordnet sind.

6. System (10) nach Anspruch 5, **dadurch gekennzeichnet dass** die zwei oder mehreren inflatierbaren Elemente (20; 21) unabhängig voneinander oder zusammen inflatiert werden können.

7. System (10) nach einem der vorstehenden Ansprüche, **dadurch gekennzeichnet dass** es ferner zumindest ein röntgendichtes Markerelement auf zumindest einem der Folgenden aufweist: Prothese (30), äußerer Pusherschlauch (12), innerer Trägerschlauch (16).

8. System (10) nach einem der vorstehenden Ansprüche, **dadurch gekennzeichnet dass** es ferner einen Führungsdraht aufweist, der innerhalb des inneren Schlauchs (16) in einem Führungsdrahtlumen (32) platzierbar ist.

9. System (10) nach einem der vorstehenden Ansprüche, **dadurch gekennzeichnet dass** es ferner auf sowohl dem proximalen Abschnitt (18) des inneren Trägerschlauchs (16) als auch auf dem äußeren Pusherschlauch (12) jeweils ein Griffelement (26; 28) aufweist.

10. System (10) nach Anspruch 9, **dadurch gekennzeichnet dass** die Griffelemente (26; 28) in Bezug aufeinander fixiert und wieder voneinander gelöst werden können.

11. System (10) nach einem der vorstehenden Ansprüche, **dadurch gekennzeichnet dass** es ferner eine schlauchförmige Gallengang- oder Pankreasgang-Prothese (30) aufweist, die auf dem inneren Schlauch (16) positionierbar ist.

## Revendications

1. Système (10) pour déployer et/ou rétracter une prothèse tubulaire de conduit biliaire ou pancréatique dans/à partir d'un canal du corps d'un mammifère, le système (10) comprenant:
- un tube de poussée extérieur allongé (12) présentant une extrémité proximale (13) et une extrémité distale (14),
- un tube de support intérieur allongé (16) présentant une partie de tube proximale (18), distale (17) et intermédiaire (19), le tube de support intérieur (16) étant partiellement disposé à l'intérieur du tube de poussée extérieur (12) et étant mobile par rapport au tube de poussée extérieur (12), dans lequel le tube de poussée extérieur (12) et le tube de support intérieur (16) sont disposés de façon concentrique l'un par rapport à l'autre,
**caractérisé en ce que** la partie intermédiaire (19) du tube de support intérieur (16) comprend au moins un élément gonflable (20) qui peut passer d'un état gonflé à un état dégonflé, et qui a la forme d'un tube court, avec les deux extrémités du tube court qui sont montées sur la partie intermédiaire (19) du tube intérieur, dans lequel, pour introduire une prothèse à l'intérieur d'un canal ou pour rétracter une prothèse, l'élément gonflable (20) se trouve à l'état gonflé afin de fixer la prothèse par rapport au tube de support intérieur (16), et dans lequel, pour libérer la prothèse dans le canal à une position souhaitable, l'élément gonflable (20) peut être actionné pour être converti à l'état dégonflé.

2. Système (10) selon la revendication 1, dans lequel l'élément gonflable (20) est un ballon.

3. Système (10) selon la revendication 2, dans lequel, à l'état gonflé, le ballon présente une forme allongée.

4. Système (10) selon l'une quelconque des revendications 1 à 3, **caractérisé en ce qu'**un premier élément gonflable (20) est agencé sur le tube de support intérieur (16), de telle sorte qu'il soit positionné sensiblement à proximité de l'extrémité distale (14) du tube de poussée extérieur (12).

5. Système (10) selon la revendication 4, **caractérisé en ce que** le tube de support intérieur (16) comprend un deuxième ou plusieurs autres élément(s) gonflable(s) (21), le deuxième ou les autres élément(s) gonflable(s) (22) étant agencé(s) sur la partie distale (17) du tube de support intérieur (16).

6. Système (10) selon la revendication 5, **caractérisé en ce que** les deux ou plus de deux éléments gonflables (20; 21) peuvent être gonflés indépendamment ou ensemble.

7. Système (10) selon l'une quelconque des revendications précédentes, **caractérisé en ce qu'**il comprend en outre au moins un élément de marqueur radio-opaque sur au moins un des éléments suivants: la prothèse (30), le tube de poussée extérieur (12) et le tube de support intérieur (16).

8. Système (10) selon l'une quelconque des revendications précédentes, **caractérisé en ce qu'**il comprend en outre un fil de guidage qui peut être situé à l'intérieur du tube intérieur (16) dans une lumière de fil de guidage (32).

9. Système (10) selon l'une quelconque des revendications précédentes, **caractérisé en ce qu'**il comprend en outre un élément de saisie (26; 28) sur la partie proximale (18) à la fois du tube de support intérieur (16) et du tube de poussée extérieur (12), respectivement.

10. Système (10) selon la revendication 9, **caractérisé en ce que** les éléments de saisie (26; 28) peuvent être fixé l'un par rapport à l'autre et détachés l'un de l'autre à nouveau.

11. Système (10) selon l'une quelconque des revendications précédentes, **caractérisé en ce qu'**il comprend en outre une prothèse tubulaire de canal biliaire ou pancréatique (30) qui peut être positionnée sur le tube intérieur (16).
